# EUROPEAN PATENT SPECIFICATION

(11) **EP 2 014 266 B1**
(45) Date of publication and mention of the grant of the patent: **16.12.2009**
(21) Application number: 08102437.4
(22) Date of filing: 10.03.2008
(51) Int. Cl.: A61F 9/007, A61M 1/00

(54) **Method of priming a surgical system**
Verfahren zur Vorbereitung eines chirurgischen Systems
Procédé pour l'amorce d'un système chirurgical

(30) Priority: 09.07.2007 US 774633
(43) Date of publication of application: 14.01.2009
(73) Proprietor: Alcon, Inc., 6331 Hünenberg (CH)
(72) Inventor: Davis, Sherman G, Laguna Niguel, CA 92677 (US); Sorensen, Gary P., Laguna Niguel, CA 92677 (US)
(74) Representative: Hanna, Peter William Derek

(56) References cited:
- EP-A- 0 717 970
- EP-A- 1 310 267
- WO-A-2007/001859
- WO-A-2007/001929

## Description

### Background of the Invention

This invention relates generally to the field of cataract surgery and more particularly to a priming method for use with a phacoemulsification system.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquefies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

The operative part of the handpiece is a centrally located, hollow resonating bar or horn directly attached to a set of piezoelectric crystals. The crystals supply the required ultrasonic vibration needed to drive both the horn and the attached cutting tip during phacoemulsification and are controlled by the console. The crystal/horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal-driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of-the irrigating sleeve and the cutting tip.

Prior to use in surgery, the various handpieces, tubings and fluid management cassettes all need to be purged of air or primed. During the priming stage, current phacoemulsification systems also run a system diagnostic step to test for leaks or blockages in the irrigation/aspiration system. Preferably the initial priming/diagnostic procedure is done prior to installation of the surgical handpiece by connecting the irrigation and aspiration fittings or lines together; however some equipment may perform this prime/diagnostic procedure with the surgical handpiece installed. During the diagnostic step, the system pump is activated to generate a certain vacuum in the aspiration line, generally around 400 mm Hg or less. If the system is not able to reach the desired vacuum level, this indicates to the system that there is a leak somewhere in the aspiration system, and the system will provide a warning for the operator. On the other hand, inability to release previously built vacuum indicates that there is a blockage in the system, such as a kink in one of the tubings.

After connecting the surgical (phaco) handpiece, an additional diagnostic test is done to verify an adequate fluid flow through the surgical handpiece. Current phacoemulsification systems typically use a small rubber test chamber that fits over the cutting tip and sleeve to close the fluid path. During this test an excessive vacuum level in the aspiration line for a given pump speed would indicate a flow restriction in the fluidic path. Also, a manual check can be performed by the user to ensure that the closed system is filled and pressurized upon test completion. A deflated test chamber, for example, indicates an irrigation flow restriction.

While this priming and diagnostic system procedure is effective, it is unable to remove all of the air from within the fluid system. Pockets of air remain entrained within the various passages due to fluid path geometry and/or surface tension. This residual entrained air adds to system compliance and has a deleterious effect on overall system performance.

Therefore, a need continues to exist for a method of priming surgical systems that helps to purge air from the system.

A method according to the preamble of claim 1 is taught by WO 2007/001929.

### Brief Summary of the Invention

The present invention improves upon the prior art by providing a method of priming a surgical system that includes pulling a very high vacuum and then cycling fluid through the system as a fluid pulse, in accordance with claims which follow. These steps can be repeated a number of times to help ensure that residual entrained air is removed from the system.

Another objective of the present invention is to provide a more reliable method for priming a surgical system that helps to purge entrained air from the system.

These and other advantages and objectives of the present invention will become apparent from the detailed description and claims that follow.

### Brief Description of the Drawings

FIG. 1 is a block diagram of a surgical system capable of practicing the method of the present invention.
FIG. 2 is a block diagram of a prior art second surgical system.

### Detailed Description of the Invention

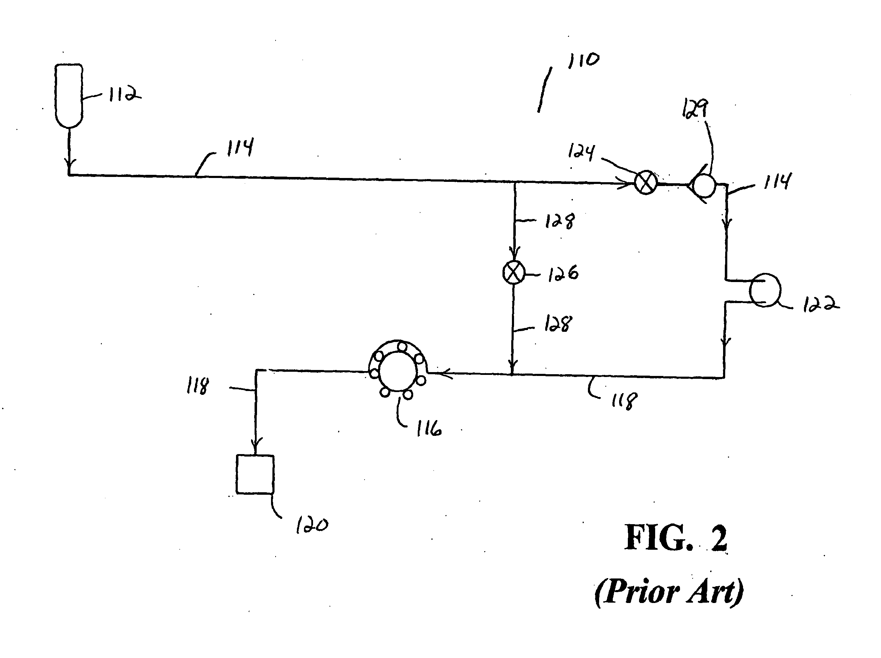

As best seen in FIG. 2, prior art surgical system 110 includes irrigation fluid source 112, irrigation fluid supply line 114, pump 116, aspiration line 118 and drain bag120. During initial set up and priming, irrigation/aspiration junction 122 is formed in the manner described above. Irrigation fluid flow may flow from irrigation fluid source 112 to drain bag 120 because irrigation fluid source 112 is pressurized, but generally speaking, fluid flow through irrigation line 114, irrigation/aspiration junction 122, aspiration line 118 and into drain bag 120 is caused by the operation ofpump 116. The flow of irrigation fluid in irrigation fluid supply line 114 is controlled by irrigation valve 124 and check valve 129. The vacuum in aspiration line 118 can be reduced or vented by operation of vent valve 126 that is placed in vent line 128. Vent line 128 fluidly communicates between irrigation supply line 114 and aspiration line 118 between pump 116 and irrigation/aspiration junction 122. System 110 is primed by closing vent valve126, opening irrigation valve 124 and operating pump 116 to produce a vacuum of generally around 500 mm Hg or more. Once irrigation line 114, aspiration line 118 and pump 116 are primed, vent valve 26 is opened to prime vent line 128.

In order to purge residual entrained air from system 110, irrigation valve 124 and vent valve 126 are closed, and pump 116 is operated to produce a high vacuum of around 600 mm Hg or greater in aspiration line 118. Irrigation valve 124 is then opened, producing a sudden high flow pulse from bottle 112 through valve 124, irrigation supply line 114, irrigation/aspiration junction 122 and aspiration line 118. Irrigation valve 124 is closed and pump 116 is once again operated to produce a high vacuum in aspiration line 118. Vent valve 126 is then opened producing a sudden high flow pulse from bottle 112 through irrigation line 114, valve 126 and vent line 128.

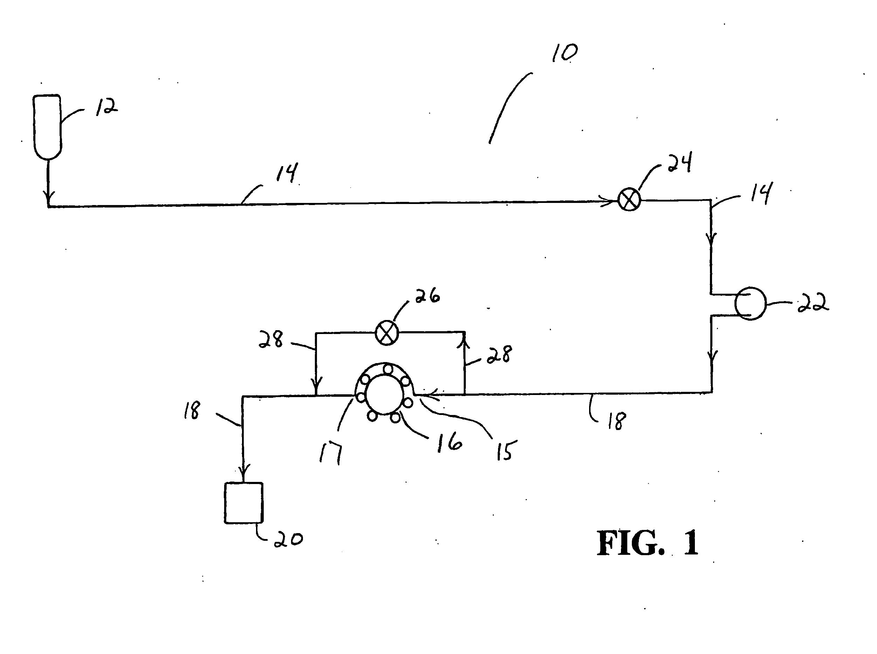

As best seen in FIG. 1 surgical system 10 of the present invention generally includes irrigation fluid source 12, irrigation fluid supply line 14, pump 16, aspiration line 18 and drain bag 20. During initial set up and priming, for example, irrigation/aspiration junction 22 is formed by connecting irrigation line 14 and aspiration line 18 together directly or through a handpiece with a test chamber. Irrigation fluid flow may flow from irrigation fluid source 12 to drain bag 20 because irrigation fluid source 12 is pressurized, but generally speaking, fluid flow through irrigation line 14, irrigation/aspiration junction 22, aspiration line 18 and into drain bag 20 is caused by the operation of pump 16. The flow of irrigation fluid in irrigation fluid supply line 14 is controlled by irrigation valve 24. The vacuum in aspiration line 18 between pump 16 and irrigation/aspiration junction 22 can be reduced or vented by operation of vent valve 26 that is placed in vent line 28. Vent line 28 fluidly communicates with both input side 15 and output side 17 of pump 16. System 10 is primed by closing vent valve 26, opening irrigation valve 24 and operating pump 16 to produce a vacuum of generally around 400 mm Hg or less. Once irrigation line 14, aspiration line 18 and pump 16 are primed, vent valve 26 is opened to prime vent line 28.

Residual entrained air may be purged from difficult to prime passages within system 10 in the following manner. Irrigation valve 24 and vent valve 26 are closed, and pump 16 is operated to produce a high vacuum (*e.g.,* at least 500 nun Hg and more preferably around 600 nun Hg or greater) in aspiration line 18. Irrigation valve 24 is then opened, producing a sudden high flow pulse from bottle 12 through valve 24, irrigation supply line 14, irrigation/aspiration junction 22, and aspiration line 18. This sudden high flow condition helps to dislodge trapped air and adherent air bubbles within difficult to prime irrigation/aspiration passages, and pushes the air into the main flow path. Once the air is in the main flow path it is more easily removed from the system by operation of pump 16. Irrigation valve 24 is closed and pump 16 is once again operated to produce a high vacuum in aspiration line 18. Vent valve 26 is then opened producing a sudden high flow pulse from drain bag 20 through aspiration line 18 between drain bag 20 and output side 17 of pump 16, vent line 28, vent valve 26 and aspiration line 18 between input side 15 of pump 16 and irrigation/aspiration junction 22. This sudden high flow condition helps to dislodge trapped air and adherent air bubbles within difficult to prime vent path passages and pushes the air into the main flow path. Once in the main flow path, air can more easily be removed from the system by operation of pump 16. The above sequence may be repeated several times, if desired.

This description is given for purposes of illustration and explanation. It will be apparent to those skilled in the relevant art that changes and modifications may be made to the invention described above without departing from its scope.

## Claims

1. A method for priming a surgical system (10), the surgical system having an irrigation path (14) and an aspiration path (18), comprising the steps of:
a) connecting the irrigation path to a source (12) of irrigation fluid, the irrigation path having a first valve (24);
b) connecting the aspiration path to a pump (16), the pump having an inlet side (15) and an outlet side (17), the pump further capable of producing a vacuum in the aspiration path;
c) connecting the pump inlet side (15) to the pump outlet side (17) through a second valve (26);
said method being **characterized by**
d) closing the first and the second valves (24,26);
e) operating the pump (16) to produce a vacuum in the aspiration path (18); and
f) opening the first valve (24) to produce a fluid pulse from the source (12) of irrigation fluid through the aspiration path (18) to the pump (16).

2. The method of claim 1, further comprising repeating steps d) through f) a sufficient number of times to dislodge trapped air and adherent air bubbles in the aspiration path (18) and/or the irrigation path (14).

3. The method of claim 1, comprising the further steps of:
g) closing the first valve (24);
h) operating the pump (16) to produce a vacuum in the aspiration path (18); and
i) opening the second valve (26) to produce a fluid pulse from an aspiration drain (20) through the aspiration path (18) and a vent path (28) to the pump(16).

4. The method of claim 2, further comprising of repeating steps g) through i) a sufficient number of times to dislodge trapped air and adherent air bubbles in the vent path (28).

6. The method of claim, wherein the pump (16) produces a vacuum in the aspiration path (18) of 600 mm Hg or greater.

5. The method of any one of claims 1 to 4, wherein the pump (16) produces a vacuum in the aspiration path (18) of 500 mm Hg or greater.

## Patentansprüche

1. Verfahren zum Vorfüllen eines chirurgischen Systems (10), wobei das chirurgische System mit einem Spül- bzw. Irrigationsweg (14) und einem Ansaug- bzw. Aspirationsweg (18) die Schritte aufweist:
a) Verbinden des Irrigationsweges mit einer Quelle (12) für ein Spül- bzw. Irrigationsfluid, wobei der Irrigationsweg ein erstes Ventil (24) hat;
b) Verbinden des Aspirationsweges mit einer Pumpe (16), wobei die Pumpe eine Einlassseite (15) und eine Auslassseite (17) hat und ferner in der Lage ist, einen Unterdruck im Aspirationsweg zu erzeugen;
c) Verbinden der Pumpeneinlassseite (15) mit der Pumpenauslassseite (17) über eine zweites Ventil (26);
wobei das Verfahren **gekennzeichnet ist durch**:
d) Schließen des ersten und zweiten Ventils (24, 26);
e) Inbetriebnehmen der Pumpe (16), um einen Unterdruck im Aspirationsweg (18) zu erzeugen; und
f) Öffnen des ersten Ventils (24), um einen Fluidimpuls von der Quelle (12) des Irrigationsfluids über den Aspirationsweg (18) zur Pumpe (16) zu erzeugen.

2. Verfahren nach Anspruch 1, das ferner die ausreichend häufige Wiederholung der Schritte d) bis f) aufweist, um eingeschlossene Luft und anhaftende Luftblasen im Aspirationsweg (18) und/oder im Irrigationsweg (14) zu beseitigen.

3. Verfahren nach Anspruch 1, das ferner die Schritte aufweist:
g) Schließen des ersten Ventils (24);
h) Inbetriebnehmen der Pumpe (16), um einen Unterdruck im Aspirationsweg (18) zu erzeugen; und
i) Öffnen des zweiten Ventils (26), um einen Fluidimpuls von einer Aspirationsdrainage (20) über den Aspirationsweg (18) und einen Entlüftungsweg (28) zur Pumpe (16) zu erzeugen.

4. Verfahren nach Anspruch 2, das ferner die ausreichend häufige Wiederholung der Schritte g) bis i) aufweist, um eingeschlossene Luft und anhaftende Luftblasen im Entlüftungsweg (28) zu beseitigen.

5. Verfahren einem der Ansprüche 1 bis 4, bei dem die Pumpe (16) einen Unterdruck von 500 mm Hg oder mehr im Aspirationsweg (18) erzeugt.

6. Verfahren nach Anspruch 5, bei dem die Pumpe (16) einen Unterdruck von 600 mm Hg oder mehr im Aspirationsweg (18) erzeugt.

## Revendications

1. Procédé pour amorcer un système chirurgical (10), le système chirurgical ayant un trajet d'irrigation (14) et un trajet d'aspiration (18), comprenant les étapes consistant à :
a) relier le trajet d'irrigation à une source (12) de fluide d'irrigation, le trajet d'irrigation ayant une première soupape (24) ;
b) relier le trajet d'aspiration à une pompe (16), la pompe ayant un côté entrée (15) et un côté sortie (17), la pompe pouvant en outre produire un vide dans le trajet d'aspiration ;
c) relier le côté entrée de pompe (15) au côté sortie de pompe (17) à travers une seconde soupape (26),
ledit procédé étant **caractérisé par** :
d) la fermeture des première et seconde soupapes (24, 26) ;
e) l'actionnement de la pompe (16) pour produire un vide dans le trajet d'aspiration (18) ; et
f) l'ouverture de la première soupape (24) pour produire une impulsion de fluide provenant de la source (12) de fluide d'irrigation à travers le trajet d'aspiration (18) vers la pompe (16).

2. Procédé selon la revendication 1, comprenant de plus la répétition des étapes d) à f) un nombre suffisant de fois pour déplacer l'air piégé et les bulles d'air adhérentes dans le trajet d'aspiration (18) et/ou le trajet d'irrigation (14).

3. Procédé selon la revendication 1, comprenant en outre des étapes consistant à :
g) fermer la première soupape (24) ;
h) actionner la pompe (16) pour produire un vide dans le trajet d'aspiration (18) ; et
i) ouvrir la seconde soupape (26) pour produire une impulsion de fluide à partir d'une purge d'aspiration (20) à travers le trajet d'aspiration (18) et un trajet de ventilation (28) vers la pompe (16).

4. Procédé selon la revendication 2, comprenant en outre la répétition des étapes g) à i) un nombre suffisant de fois pour déplacer l'air piégé et des bulles d'air adhérentes dans le trajet de ventilation (28).

5. Procédé selon l'une quelconque des revendications 1 à 4, dans lequel la pompe (16) produit un vide dans le trajet d'aspiration (18) de 500 mm Hg ou plus.

6. Procédé selon la revendication 5, dans lequel la pompe (16) produit un vide dans le trajet d'aspiration (18) de 600 mm Hg ou plus.
